# EUROPÄISCHE PATENTANMELDUNG

(11) **EP 2 329 777 A2**
(43) Veröffentlichungstag der Anmeldung: **08.06.2011**
(21) Anmeldenummer: 10010010.6
(22) Anmeldetag: 21.09.2010
(51) Int. Cl.: A61B 17/50, A61B 19/00

(54) **Vorrichtung zur Entfernung von Zecken aus einem menschlichen oder tierischen Körper**

(30) Priorität: 01.12.2009 DE 202009016207 U
(71) Anmelder: Hepla-Kunststofftechnik GmbH & Co. KG, 34576 Homberg (DE)
(72) Erfinder: Fischer, Knuth, 34560 Fritzlar (DE)
(74) Vertreter: Walther, Walther & Hinz GbR

(57) **Zusammenfassung**

Die Erfindung betrifft eine Vorrichtung (1) zur Entfernung von Zecken aus einem menschlichen oder tierischen Körper, umfassend einen Zeckenentferner (12) zum Erfassen der Zecke, wobei die Vorrichtung (1) örtlich über dem Zeckenentferner (12) eine Lupe (25) aufweist.

## Beschreibung

Die vorliegende Erfindung betrifft eine Vorrichtung zur Entfernung von Zecken aus einem menschlichen oder tierischen Körper.

Zeckenzangen oder auch Zeckenkarten zum Entfernen von Zecken sind aus dem Stand der Technik hinreichend bekannt. Bei Zeckenzangen handelt es sich im Wesentlichen um pinzettenartige Gebilde, die den Zeckenkörper kurz oberhalb der Hautoberfläche erfassen und durch Anheben der Pinzette die Zecke aus der Haut entfernen.

Zeckenkarten zeichnen sich durch ein Format ähnlich einer Scheckkarte aus, die an einer Seite oder an einer Kante eine trichterförmige Aussparung aufweist, mit welcher die Karte unter die Zecke geschoben wird, um dann die Zecke quasi von der Haut abzuscheren. Insbesondere die Zeckenarten funktionieren durchaus befriedigend.

Allerdings ist insbesondere dann, wenn die Zecke noch sehr klein ist, die Zecke auf der Haut häufig schwierig auszumachen. Dies gilt insbesondere dann, wenn die Stelle auf der Haut, von der die Zecke entfernt werden soll, mehr oder weniger behaart ist. Das heißt, es ist in einem solchen Fall ratsam, die Zecke bzw. die Umgebung der Zecke mit einer Lupe zu betrachten, um genau eruieren zu können, an welcher Stelle die Zeckenkarte angesetzt werden soll. In diesem Zusammenhang ist insofern der Einsatz einer Lupe durchaus ratsam.

Die der Erfindung zugrunde liegende Aufgabe besteht demzufolge darin, eine Vorrichtung zur Entfernung von Zecken aus einem menschlichen oder tierischen Körper bereitzustellen, mit welcher die Person, die zur Entfernung der Zecke eingesetzt wird, in der Lage ist, beim Vorgang der Entfernung der Zecke genau beobachten zu können, wie die Zeckenkarte mit der trichterförmigen Öffnung an der Zecke angesetzt wird.

Eine Vorrichtung, die diese Aufgabe erfüllt, weist erfindungsgemäß einen Zeckenentferner, z. B. in Form einer trichterförmigen Aussparung zum Erfassen der Zecke auf, wobei die Vorrichtung örtlich über dem Zeckenentferner eine Lupe aufweist. Im Einzelnen umfasst diese Vorrichtung zwei, insbesondere durch ein Scharnier schwenkbar miteinander verbundene Aufnahmeglieder, wobei das eine Aufnahmeglied den Zeckenentferner und das andere Aufnahmeglied die Lupe aufweist, wobei im aufeinander zugeschwenkten Zustand der beiden Aufnahmeglieder die Lupe sich über dem Zeckenentferner z. B. in Form der trichterförmigen Aussparung befindet. Hieraus wird deutlich, dass der Vorgang des Entfernens der Zecke durch die Lupe betrachtet werden kann, da - wie bereits ausgeführt - sich die Lupe unmittelbar über dem Zeckenentferner, mithin der trichterförmigen Aussparung befindet. Es ist also nicht erforderlich, dass eine gesonderte Lupe bereitgehalten wird, und es ist auch nicht erforderlich, dass der Vorgang des Betrachtens der Zecke und der Vorgang des eigentlichen Entfernens der Zecke zwei getrennte Vorgänge sind, vielmehr ist - wie bereits ausgeführt - die Möglichkeit eröffnet, den Vorgang des Entfernens der Zecke durch die Lupe zu beobachten.

Vorteilhafte Merkmale ergeben sich aus den Unteransprüchen.

So ist insbesondere vorgesehen, dass die beiden Aufnahmeglieder durch ein Filmscharnier miteinander verbunden sind. Zeckenkarten werden häufig auf Wanderungen mitgenommen. Man ist naturgemäß bestrebt, auf Wanderungen den Platzbedarf für solche Gegenstände und auch das Gewicht möglichst gering zu halten. Eine solche Vorrichtung, wie sie Gegenstand der Erfindung ist, wiegt im Prinzip praktisch nichts. Im auseinandergeklappten Zustand der beiden Aufnahmeglieder ist die Vorrichtung zur Entfernung von Zecken im Wesentlichen flach, ähnlich einer Karte und kann in die Brieftasche gesteckt werden. Erst durch das Aufeinanderzuklappen der beiden Aufnahmeglieder über das die beiden Aufnahmeglieder verbindende Filmscharnier wird nunmehr ein räumlicher Gegenstand geschaffen, der die Entfernung der Zecke ermöglicht, bei gleichzeitiger Betrachtung des Entfernungsvorgangs.

Um die Handhabung der erfindungsgemäßen Vorrichtung weiterhin zu optimieren, ist vorgesehen, dass die beiden Aufnahmeglieder im aufeinander zugeschwenkten Zustand miteinander in Verbindung stehen, sodass sich ein im Wesentlichen stabiles dreieckförmiges Gebilde ergibt. In diesem Zusammenhang ist insbesondere vorgesehen, dass das eine Aufnahmeglied seitlich hochklappbare Wandungen aufweist, die in Eingriff mit dem anderen Aufnahmeglied bringbar sind, sodass sich - wie bereits erläutert - der dreieckförmige Körper in der Seitenansicht ergibt. Zur Verbindung der beiden Aufnahmeglieder im Seitenwandbereich weist das eine Aufnahmeglied Rastnasen auf, die in entsprechende Ausnehmungen des anderen Aufnahmegliedes einrastbar sind.

Die beiden Aufnahmeglieder der Vorrichtung zur Entfernung von Zecken sind insbesondere kartenartig ausgebildet und bestehen vorzugsweise aus Kunststoff, wobei die Lupe nach einem besonderen Merkmal der Erfindung in das andere Aufnahmeglied eingespritzt sein kann. Das heißt, die Herstellung der erfindungsgemäßen Vorrichtung ist besonders einfach und insofern ist eine solche Vorrichtung auch preiswert herstellbar, was die Möglichkeit eröffnet, eine solche Vorrichtung nach jedem Einsatz aus hygienischen Gründen unmittelbar zu entsorgen. Denkbar ist allerdings auch die einclipsbare Aufnahme einer separaten Lupe durch das Aufnahmeglied. Hierzu besitzt das Aufnahmeglied zangenförmig angeordnete Arme, die die Lupe oder die Linse klemmbar erfassen.

Anhand der Zeichnungen wird die Erfindung beispielhaft näher erläutert.

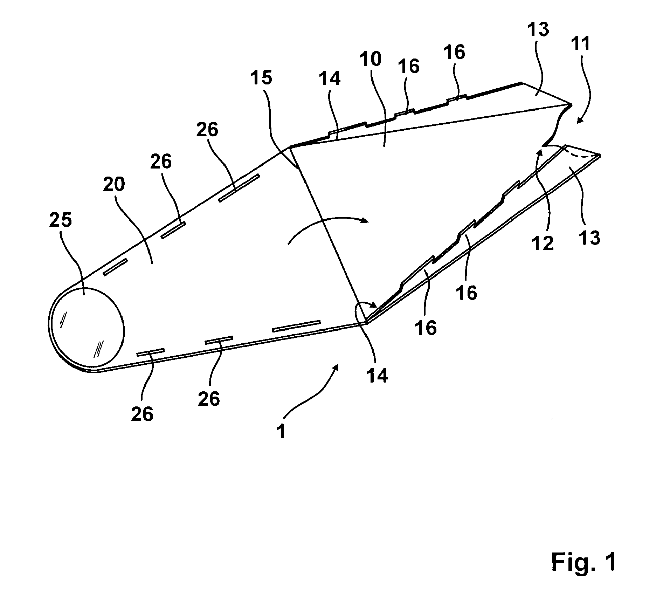

- Figur 1: zeigt die Vorrichtung im auseinandergeklappten Zustand;
- Figur 2: zeigt die Vorrichtung im zusammengebauten Zustand;
- Figur 3: zeigt die Vorrichtung gemäß Figur 1 mit einclispbarer Lupe.

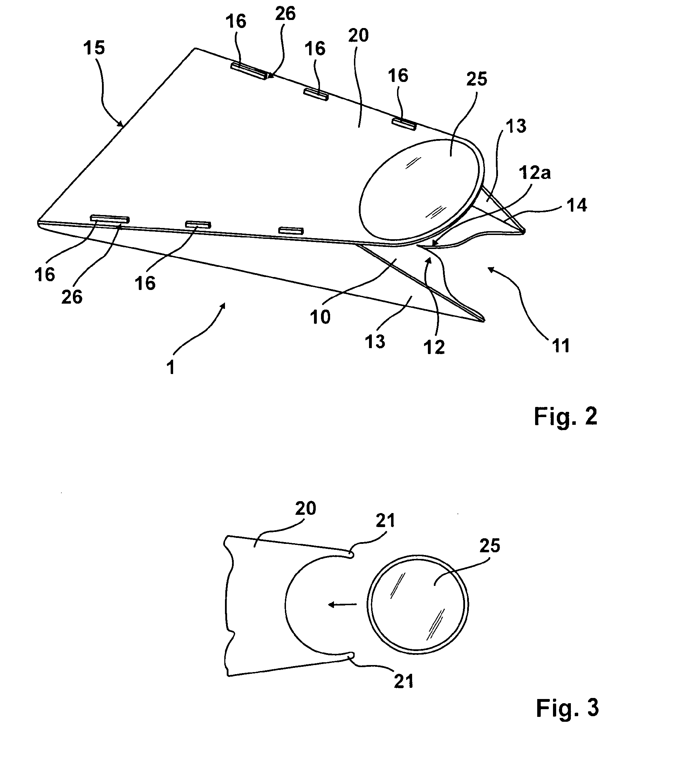

Die insgesamt mit 1 bezeichnete Vorrichtung umfasst das eine erste Aufnahmeglied 10 und das andere zweite Aufnahmeglied 20. Die beiden Aufnahmeglieder 10 und 20 sind durch das Filmscharnier 15 miteinander verbunden. Das eine erste Aufnahmeglied besitzt am freien Ende (Pfeil 11) die trichterförmige Aussparung 12, die als Werkzeug zum Entfernen der Zecke fungiert. Die trichterförmige Aussparung mündet in einer sich stetig verjüngenden Spitze 12a, wie dies insbesondere aus Figur 2 erkennbar ist. Das eine Aufnahmeglied 10 weist des Weiteren seitlich angeordnete hochklappbare Wandungen 13 auf, die ebenfalls über Filmscharniere 14 mit dem Aufnahmeglied 10 in Verbindung stehen. Die hochklappbaren Wandungen besitzen an ihrer dem anderen Aufnahmeglied zugewandten Seite Rastnasen 16, die mit entsprechenden Ausnehmungen 26 in dem anderen Aufnahmeglied 20 einrastbar fixierbar sind. Das andere Aufnahmeglied 20 weist darüber hinaus die Lupe 25 auf, die im zusammengeklappten Zustand der beiden Aufnahmeglieder 10, 20 (Figur 2) sich unmittelbar über der trichterförmigen Aussparung 12 befindet.

Die Entfernung der Zecke erfolgt nun dadurch, dass die Vorrichtung im zusammengebauten Zustand gemäß Figur 2 mit der trichterförmigen Aussparung 12 unter die Zecke geschoben wird und hierbei die Zecke abgeschert oder auch abgehoben wird.

Bei der Ausbildung gemäß Figur 3 weist das Aufnahmeglied 20 zangenförmig angeordnete Arme 21 auf, die die Lupe klemmbar erfassen.

## Patentansprüche

1. Vorrichtung (1) zur Entfernung von Zecken aus einem menschlichen oder tierischen Körper, umfassend einen Zeckenentferner (12) zum Erfassen der Zecke, wobei die Vorrichtung (1) örtlich über dem Zeckenentferner (12) eine Lupe (25) aufweist.

2. Vorrichtung nach Anspruch 1,
**gekennzeichnet durch**
zwei, insbesondere ein Scharnier (15) schwenkbar miteinander verbundene Aufnahmeglieder (10, 20), wobei das eine Aufnahmeglied (10) den Zeckenentferner (12) und das andere Aufnahmeglied (20) die Lupe (25) aufweist, wobei im aufeinander zugeschwenkten Zustand der beiden Aufnahmeglieder (10, 20) die Lupe (25) sich über dem Zeckenentferner (12) befindet.

3. Vorrichtung nach Anspruch 1,
**dadurch gekennzeichnet,**
**dass** die beiden Aufnahmeglieder (10, 20) durch ein Filmscharnier (15) miteinander in Verbindung stehen.

4. Vorrichtung nach einem der voranstehenden Ansprüche,
**dadurch gekennzeichnet,**
**dass** die beiden Aufnahmeglieder (10, 20) im aufeinander zugeschenkten Zustand miteinander in Verbindung stehen.

5. Vorrichtung nach einem der voranstehenden Ansprüche,
**dadurch gekennzeichnet,**
**dass** die beiden Aufnahmeglieder (10, 20) im aufeinander zugeschwenkten Zustand durch Seitenwände (13) miteinander in Verbindung stehen.

6. Vorrichtung nach einem der voranstehenden Ansprüche,
**dadurch gekennzeichnet,**
**dass** die Aufnahmeglieder (10, 20) kartenartig ausgebildet sind.

7. Vorrichtung nach einem der voranstehenden Ansprüche,
**dadurch gekennzeichnet,**
**dass** die Aufnahmeglieder (10, 20) aus Kunststoff ausgebildet sind.

8. Vorrichtung nach einem der voranstehenden Ansprüche,
**dadurch gekennzeichnet,**
**dass** die Lupe (25) in das Aufnahmeglied (20) eingespritzt ist.

9. Vorrichtung nach einem der voranstehenden Ansprüche,
**dadurch gekennzeichnet,**
**dass** der Zeckenentferner (12) in dem kartenartigen Aufnahmeglied (10) in der Draufsicht trichterförmig ausgebildet ist.

10. Vorrichtung nach einem der voranstehenden Ansprüche,
**dadurch gekennzeichnet,**
**dass** das eine Aufnahmeglied (10) hochklappbare Seitenwände (13) aufweist, die in Eingriff mit dem anderen Aufnahmeglied (20) bringbar sind, sodass sich ein in der Seitenansicht dreieckförmiger Körper ergibt.

11. Vorrichtung nach einem der voranstehenden Ansprüche,
**dadurch gekennzeichnet,**
**dass** die Wandungen (13) Rastnasen (16) aufweisen, die in entsprechende Ausnehmungen (26) des anderen Aufnahmegliedes (20) einrastbar sind.

12. Vorrichtung nach einem der voranstehenden Ansprüche,
**dadurch gekennzeichnet,**
**dass** das andere Aufnahmeglied (20) zangenförmig angeordnete Arme (21) zur Aufnahme der Lupe (25) aufweist.
